# EUROPÄISCHE PATENTANMELDUNG

(11) **EP 2 942 723 A1**
(43) Veröffentlichungstag der Anmeldung: **11.11.2015**
(21) Anmeldenummer: 14168903.4
(22) Anmeldetag: 19.05.2014
(51) Int. Cl.: G06F 19/00

(54) **Verfahren und System zum Betreiben einer elektronischen Patientenakte mit Patientendaten eines Patienten**

(30) Priorität: 09.05.2014 EP 14167790
(71) Anmelder: Health CoCon GmbH, 69190 Walldorf (DE)
(72) Erfinder: Hammer, Wolfgang, 69168 Wiesloch (DE); Bächle, Siegfried, 69181 Leimen (DE); Feldmann, Patric, 76344 Eggenstein-Leopoldshafen (DE); Feldmann, Frederic, 76344 Eggenstein-Leopoldshafen (DE)
(74) Vertreter: Patent- und Rechtsanwälte Ullrich & Naumann

(57) **Zusammenfassung**

Ein Verfahren zum Betreiben einer elektronischen Patientenakte mit, vorzugsweise patientenspezifische bzw. -bezogene Informationen umfassenden, Patientendaten eines Patienten, wobei die Patientendaten Text-, Bild-, Audio- und/oder Videodaten umfassen, wobei die Patientendaten in einem Datenspeicher unter der Kontrolle des Patienten gespeichert werden, wobei die Patientendaten über ein mobiles Datenkommunikationsgerät gesammelt, bereitgestellt und/oder verwaltet werden, und wobei der Patient einen Zugriff auf die elektronische Patientenakte, insbesondere zur Sammlung, Bereitstellung und/oder Verwaltung der Patientendaten, über das mobile Datenkommunikationsgerät steuert und/oder autorisiert. Des Weiteren ist ein entsprechendes System zum Betreiben einer elektronischen Patientenakte angegeben.

## Beschreibung

Die Erfindung betrifft ein Verfahren und ein System zum Betreiben einer elektronischen Patientenakte mit, vorzugsweise patientenspezifische bzw. -bezogene Informationen umfassenden, Patientendaten eines Patienten.

Im Gesundheitswesen können für Patienten und Krankenkassen erhebliche Risiken und Kosten durch Doppeluntersuchungen, erhöhte Diagnoseabrechnungen und Wechselwirkungen von Medikamenten bestehen. Die Herausforderungen liegen darin begründet, dass es häufig keine gemeinsame Datengrundlage gibt, auf die behandelnde Ärzte zugreifen können. Akteure des Gesundheitswesens sind daher seit Jahren mit erheblichem Aufwand auf der Suche nach einer Lösung dieser Problematik.

Verfahren und Systeme zum Betreiben einer elektronischen Patientenakte der eingangs genannten Art sind aus der Praxis bekannt. Bei den bekannten Verfahren und Systemen, die sich mit der Erstellung einheitlicher Gesundheitsakten befassen, ist jedoch problematisch, dass diese versuchen eine zentrale Plattform bereitzustellen, auf die sämtliche Parteien vom Arzt bis hin zur Krankenkasse zugreifen können. Diese zentralisierten Systemansätze sind komplex und erfordern ein gleichzeitiges Mitmachen aller Beteiligten und sind nicht auf den Patienten ausgerichtet. Folglich sind diesen Systemen regelmäßig Grenzen gesetzt, da sie nicht mit geltenden Datenschutzgesetzen vereinbar sind.

Der vorliegenden Erfindung liegt daher die Aufgabe zugrunde, ein Verfahren und ein System zum Betreiben einer elektronischen Patientenakte mit Patientendaten eines Patienten der eingangs genannten Art derart auszugestalten und weiterzubilden, dass eine sichere und effiziente Verwendung der Patientendaten eines Patienten auf einfache Weise ermöglicht ist.

Erfindungsgemäß wird die voranstehende Aufgabe durch die Merkmale des Patentanspruchs 1 gelöst. Danach ist ein Verfahren zum Betreiben einer elektronischen Patientenakte mit, vorzugsweise patientenspezifische bzw. -bezogene Informationen umfassenden, Patientendaten eines Patienten angegeben, wobei die Patientendaten Text-, Bild-, Audio- und/oder Videodaten umfassen, wobei die Patientendaten in einem Datenspeicher unter der Kontrolle des Patienten gespeichert werden, wobei die Patientendaten über ein mobiles Datenkommunikationsgerät gesammelt, bereitgestellt und/oder verwaltet werden, und wobei der Patient einen Zugriff auf die elektronische Patientenakte, insbesondere zur Sammlung, Bereitstellung und/oder Verwaltung der Patientendaten, über das mobile Datenkommunikationsgerät steuert und/oder autorisiert.

In Bezug auf das erfindungsgemäße System ist die voranstehende Aufgabe mit den Merkmalen des Patentanspruchs 15 gelöst. Danach ist ein System zum Betreiben einer elektronischen Patientenakte mit, vorzugsweise patientenspezifische bzw. patientenbezogene Informationen umfassenden, Patientendaten eines Patienten angegeben, wobei die Patientendaten Text-, Bild-, Audio- und/oder Videodaten umfassen, wobei das System einen Datenspeicher und ein mobiles Datenkommunikationsgerät umfasst, wobei die Patientendaten in dem Datenspeicher unter der Kontrolle des Patienten speicherbar sind, wobei die Patientendaten über das mobile Datenkommunikationsgerät gesammelt, bereitgestellt und/oder verwaltet werden, und wobei ein Zugriff auf die elektronische Patientenakte, insbesondere zur Sammlung, Bereitstellung und/oder Verwaltung der Patientendaten, über das mobile Datenkommunikationsgerät durch den Patienten steuerbar und/oder autorisierbar ist.

In erfindungsgemäßer Weise ist zunächst einmal erkannt worden, dass es von ganz erheblichem Vorteil ist, wenn der Patient in den Fokus des Datenaustauschprozesses seiner eigenen Patientendaten gestellt wird, nämlich im Sinne einer elektronischen Patientenakte zur Selbstverwaltung für den Patienten. Damit hat der Patient die Möglichkeit sämtliche ihn betreffenden medizinischen Informationen zu dokumentieren und sich über seine aktuellen Behandlungen, wie zum Beispiel Impftermine oder Vorsorgeuntersuchungen auf dem Laufenden zu halten. Idealerweise hat der Patient dadurch ebenso seine sensiblen Patientendaten unter Kontrolle und die Möglichkeit seine Patientendaten entsprechend seinen Wünschen und Erfordernissen zur Verfügung zu stellen. Im Konkreten umfassen die Patientendaten Text-, Bild-, Audio- und/oder Videodaten die erfindungsgemäß in einem Datenspeicher unter der Kontrolle des Patienten gespeichert werden. Erfindungsgemäß werden die Patientendaten über ein mobiles Datenkommunikationsgerät gesammelt, bereitgestellt und/oder verwaltet, wobei der Patient einen Zugriff auf die elektronische Patientenakte, insbesondere zur Sammlung, Bereitstellung und/oder Verwaltung der Patientendaten, über das mobile Datenkommunikationsgerät steuert und/oder autorisiert. Damit kann der Patient selbst auf einfache und effiziente Weise entscheiden, wann, wo und/oder wem er seine Patientendaten freigibt bzw. zur Verfügung stellt.

Folglich ist mit dem erfindungsgemäßen Verfahren und dem erfindungsgemäßen System zum Betreiben einer elektronischen Patientenakte ein Verfahren bzw. ein System angegeben, das eine sichere und effiziente Verwendung der Patientendaten des Patienten auf einfache Weise ermöglicht.

In vorteilhafter Weise kann das mobile Datenkommunikationsgerät als Mobilfunkgerät, Smartphone, PDA, tragbares Medienabspielgerät oder dergleichen ausgebildet sein. Damit kann der Patient das mobile Datenkommunikationsgerät zur Steuerung und Autorisierung seiner Patientendaten in seiner elektronischen Patientenakte auf praktische Weise mit sich führen und zu seiner Verfügung haben.

In weiter vorteilhafter Weise können die Patientendaten unterschiedlichste und mannigfaltige Informationen über den Patienten umfassen. Zum Beispiel ist denkbar, dass die Patientendaten Arztbriefe, Diagnosen, Befunde, Krankheitsbilder, radiologische Aufnahmen, Informationen zu Allergien, Informationen zu Vorsorgeuntersuchungen, einen Impfpass, Impftermine, an dem Patienten vorgenommene Untersuchungen, behandelnde Ärzte, Medikamentenunverträglichkeiten, Informationen über derzeit eingenommene bzw. einzunehmende Medikamente und/oder Rezepte etc. umfassen können.

In Bezug auf einen komfortablen Mehrwert für den Patienten kann das mobile Datenkommunikationsgerät mit einer Erinnerungsfunktion ausgestattet sein, so dass der Patient an in der elektronischen Patientenakte hinterlegte Termine wie beispielsweise Arzttermine, Impftermine, Vorsorgeuntersuchungen etc. rechtzeitig erinnert wird.

In einer vorteilhaften Ausgestaltung kann als Datenspeicher zur Speicherung der Patientendaten ein lokal in dem mobilen Datenkommunikationsgerät angeordneter Speicher, ein vorzugsweise zentraler Server und/oder eine Cloud verwendet werden. Dabei ist es denkbar, dass die Patientendaten komplett oder auch nur teilweise lokal auf dem Datenkommunikationsgerät gespeichert sind und je nach Erfordernis ggf. benötigte Patientendaten aus der elektronischen Patientenakte abruft, die in einem vom Patienten kontrollierten Speicherbereich eines Servers oder einer Cloud hinterlegt sein können. Dabei können die Patientendaten auf dem mobilen Datenkommunikationsgerät mit den Patientendaten auf dem Server bzw. in der Cloud synchronisiert werden.

Im Hinblick auf eine möglichst einfache und effektive Sammlung von Patientendaten kann das mobile Datenkommunikationsgerät eine Scaneinrichtung umfassen, wobei mit der Scaneinrichtung Patientendaten für die Aufnahme in die elektronische Patientenakte erfasst werden. Dabei kann es sich bei der Scaneinrichtung um eine Kamera handeln, die ggf. Rezepte, Befunde, Arztberiefe etc. fotografiert, damit diese zur späteren Verfügbarkeit in die elektronische Patientenakte des Patienten aufgenommen werden.

In vorteilhafter Weise kann zur Sammlung von Patientendaten für die Aufnahme in die elektronische Patientenakte ein Informationscode, insbesondere ein QR-Code, verwendet werden, wobei der Informationscode von einer Patientendaten bereitstellenden Computervorrichtung, zum Beispiel in einer Arztpraxis, visualisiert wird und von einer von dem mobilen Datenkommunikationsgerät umfassten Scaneinrichtung zur Verarbeitung gescannt wird. Damit kann ein Arzt auf einfache und komfortable Weise dem Patienten gewünschte Informationen bzw. Patientendaten zur Verfügung stellen. Im Hinblick auf eine sichere Weitergabe von sensiblen Patientendaten kann der Informationscode dabei während eines vorgebbaren Zeitfensters visualisiert werden, so dass eine Datenweitergabe an unbefugte Personen nach Ablauf des Zeitfensters verhindert wird.

In weiter vorteilhafter Weise kann zur Bereitstellung von Patientendaten aus der elektronischen Patientenakte, die unter Kontrolle des Patienten ist, ein Informationscode, insbesondere ein QR-Code, verwendet werden, wobei der Informationscode von dem mobilen Datenkommunikationsgerät, vorzugsweise für ein vorgebbares Zeitfenster, visualisiert wird, und wobei der Informationscode von einer von einer Computervorrichtung umfassten Scaneinrichtung zur Verarbeitung gescannt wird. Somit kann ein Arzt mittels einer entsprechend konfigurierten Computervorrichtung für ihn relevante Patientendaten über seinen Patienten auf einfache und schnelle Weise direkt vom Patienten entgegennehmen, der diese mit seinem mobilen Datenkommunikationsgerät bereitstellt.

In einer vorteilhaften Ausgestaltung kann der Informationscode Patientendaten des Patienten umfassen. Somit sind die Patientendaten, die vorzugsweise patientenspezifische bzw. -bezogene Informationen umfassen, in dem Informationscode enthalten bzw. darin codiert und werden durch den Scanvorgang, zum Beispiel an den Arzt, weitergeben.

In einer weiter vorteilhaften Ausgestaltung, insbesondere im Hinblick auf die effektive Übermittlung von großen Datenmengen, kann der Informationscode eine Adressierungsinformation umfassen, wobei die Adressierungsinformation zur Adressierung eines Speicherorts mit hinterlegten Patientendaten dient, und wobei die an dem Speicherort hinterlegten Patientendaten mittels einer Datenübertragung, insbesondere über eine drahtlose Funkverbindung, abgerufen werden. Über die Adressierungsinformation können damit spezifische Patientendaten, die an dem Speicherort hinterlegt sind, abgerufen bzw. heruntergeladen werden. Als Adressierungsinformation ist hier ein Uniform Resource Identifier (URI) denkbar, der einen Identifikator darstellt und aus einer Zeichenfolge besteht, die zur Identifizierung einer abstrakten oder physischen Ressource dient. Dabei ist es denkbar, dass zum Abrufen der Patientendaten von dem Speicherort eine Internetverbindung verwendet wird. In vorteilhafter Weise ist an dem mobilen Datenkommunikationsgerät und/oder an der Computervorrichtung eine drahtlose Schnittstelle vorgesehen, so dass mittels WLAN (Wireless Local Area Network), UMTS (Universal Mobile Telecommunications System), EDGE (Enhanced Data Rates for GSM Evolution), HSDPA (High Speed Downlink Packet Access), GPRS (General Packet Radio Service), LTE (3GPP Long Term Evolution) etc. eine Verbindung zum Datenaustausch bzw. zur Datenübertragung hergestellt werden kann. Des Weiteren ist auch eine Verbindung über kurze Distanz per Funktechnik, zum Beispiel mittels Bluetooth oder NFC (Near Field Communication), zum direkten Datenaustausch zwischen dem mobilen Datenkommunikationsgerät und der Computervorrichtung denkbar.

In vorteilhafter Weise können bei der Sammlung von Patientendaten für die elektronische Patientenakte - d.h. zur Eingabe von Patientendaten bzw. zur Erweiterung der elektronischen Patientenakte mit Patientendaten - die Patientendaten in einem von der Computervorrichtung umfassten Datenspeicher als Speicherort zum Abrufen oder in einem von der Computervorrichtung bzw. vom Arzt kontrollierten Speicherbereich, vorzugsweise auf einem Server, als Speicherort zum Abrufen hinterlegt sein.

Hinsichtlich des zur Verfügungsstellens von Patientendaten, zum Beispiel an einen behandelnden Arzt, können bei der Bereitstellung von Patientendaten aus der elektronischen Patientenakte die Patientendaten in dem vom Patienten kontrollierten Datenspeicher als Speicherort zum Abrufen hinterlegt sein. Der Datenspeicher kann dabei einen lokalen Speicher auf dem mobilen Datenkommunikationsgerät oder einen Speicherbereich auf einem Server oder in einer Cloud umfassen, von dem die hinterlegten Patientendaten, vorzugsweise über eine Internetverbindung, abgerufen werden.

In vorteilhafter Weise können die zum Abrufen hinterlegten Patientendaten für ein vorgebbares Zeitfenster, vorzugsweise ab Visualisierung des Informationscodes, zum Abrufen bereitgestellt werden. Nach Ablauf des Zeitfensters oder nach dem Abrufen der hinterlegten Patientendaten kann der Zugriff auf diese gesperrt oder die hinterlegten Patientendaten selbst gelöscht werden, so dass ein weiteres Abrufen, möglichweise durch Unbefugte vermieden ist.

In einer vorteilhaften Ausgestaltung können die Patientendaten, vorzugsweise über das Scannen des Informationscodes und/oder mittels einer Datenübertragung, in verschlüsselter Form auf das mobile Datenkommunikationsgerät übertragen werden, und wobei die verschlüsselten Patientendaten mit dem Datenkommunikationsgerät nur an einem vom Patienten zuvor festgelegten geographischen Ort entschlüsselt werden. Des Weiteren ist es denkbar, dass die Eingabe einer PIN oder eines Passworts als Erfordernis zum Entschlüsseln der übertragenen Patientendaten vorgesehen ist.

In einer weiteren vorteilhaften Ausgestaltung kann das mobile Datenkommunikationsgerät vom Patienten derart eingestellt werden, dass der Zugriff auf die elektronische Patientenakte, insbesondere zur Sammlung, Bereitstellung und/oder Verwaltung der Patientendaten, über das mobile Datenkommunikationsgerät nur an einem vom Patienten zuvor festgelegten geographischen Ort durchführbar ist. Zum Beispiel könnte der Patient festlegen, dass ein Zugriff auf die elektronische Patientenakte bzw. auf deren Patientendaten nur in einer Arztpraxis, in einem Krankenhaus und/oder bei dem Patienten Zuhause möglich ist. Dazu könnte über Ortskoordinaten oder GPS-Koordinaten gesteuert werden, ob Informationen freigegeben werden dürfen oder nicht.

Im Hinblick auf eine effizienten Datenbereitstellung und/oder Datensammlung können die aus der elektronischen Patientenakte bereitzustellenden Patientendaten und/oder die für die elektronische Patientenakte zu sammelnden Patientendaten durch den Patienten über das mobile Datenkommunikationsgerät selektiert werden. Demnach kann der Patient explizit auswählen, welche seiner Patientendaten freigegeben bzw. übertragen werden.

In vorteilhafter Weise kann auf dem mobilen Datenkommunikationsgerät ein Programm installiert sein, wobei der Patient über das Programm die patientenspezifischen bzw. -bezogenen Informationen umfassenden Patientendaten verwaltet bzw. den Zugriff auf die Patientendaten steuert. In weiter vorteilhafter Weise kann das Programm auf dem mobilen Datenkommunikationsgerät zum Beispiel folgende Basisfunktionalitäten aufweisen:
- Verwaltung bzw. Zugriff auf elektronische Patientenakte mit Bild, Diagnose und Medikation
- Arztverwaltung
- Terminverwaltung (Impfungen, Vorsorge, Folgetermine, Zahnarzt etc.)
- Impf-, Allergie-, Röntgen- und Diabetespass
- Mutterpass mit Bild, Diagnose und Medikation

In vorteilhafter Weise kann das Programm eine Funktionalität für den Notfall umfassen. Im Konkreten ist eine Notfallfunktionalität denkbar, wobei durch Auslösen bzw. Aktivieren eines Notfallbuttons auf dem mobilen Datenkommunikationsgerät eine durch den Patienten zuvor definierbare Auswahl seiner Patientendaten in Form von Notfalldaten unmittelbar zur Verfügung gestellt werden, so dass in einer Notfallsituation wichtige patientenspezifische bzw. -bezogene Informationen von einem behandelnden Arzt oder einer Hilfe leistenden Person berücksichtigt werden können.

In vorteilhafter Weise könnte das Programm automatisch gestartet werden, wenn das mobile Datenkommunikationsgerät an einem zuvor von dem Patienten definierten Ort eintrifft. So könnte das Programm derart konfiguriert sein, dass das Programm automatisch startet, sobald das mobile Datenkommunikationsgerät in einem Krankenhaus eintrifft und dann über die Notfallfunktionalität vorgebbare Patientendaten zur Verfügung stellt.

Als weitere Funktionalität könnte in dem Programm ein Bezahlsystem zum einfachen Bezahlen von Rechnungen integriert sein, insbesondere zum Bezahlen von Arztrechnungen oder zum automatisierten Bezahlen von Medikamenten. Beispielsweise könnte beim Scannen eines Rezeptes über das Programm ein automatischer Bestellungs- und/oder Bezahlungsvorgang ausgelöst und durchgeführt werden.

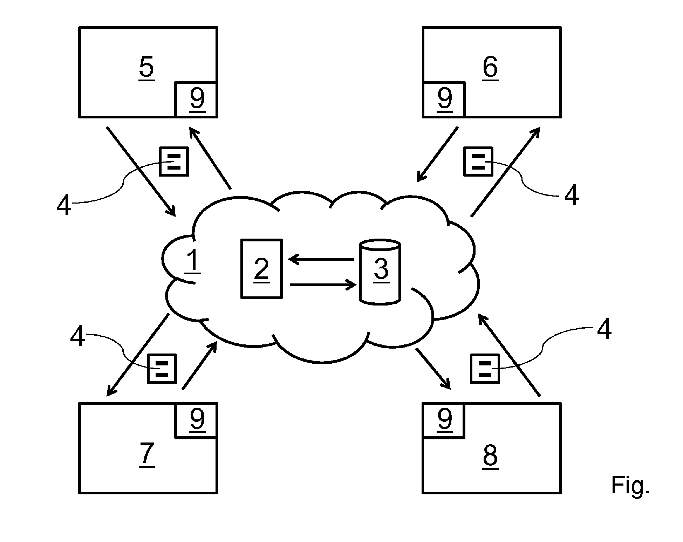

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Patentansprüche und andererseits auf die nachfolgende Erläuterung eines bevorzugten Ausführungsbeispiels der Erfindung anhand der Zeichnung zu verweisen. In Verbindung mit der Erläuterung des bevorzugten Ausführungsbeispiels der Erfindung anhand der Zeichnung werden auch im Allgemeinen bevorzugte Ausgestaltungen und Weiterbildungen der Lehre erläutert.
In der Zeichnung zeigt die einzige
- Fig.: in einer schematischen Ansicht ein Ausführungsbeispiel eines erfindungsgemäßen Verfahrens bzw. eines erfindungsgemäßen Systems.

Die einzige Fig. zeigt schematisch ein Ausführungsbeispiel eines erfindungsgemäßen Verfahrens bzw. eines erfindungsgemäßen Systems, wobei ein erheblicher Vorteil darin besteht, dass ein Patient 1 ins Zentrum des Verfahrens bzw. des Systems gesetzt wird und der Datenaustausch pragmatisch erfolgt.

Der Patient 1 steuert und/oder autorisiert den Zugriff auf seine elektronische Patientenakte bzw. auf seine Patientendaten über ein mobiles Datenkommunikationsgerät 2. Die Patientendaten bzw. die elektronische Patientenakte können/kann vollständig auf dem mobilen Datenkommunikationsgerät 2 in einem Datenspeicher hinterlegt sein. Des Weiteren kann die elektronische Patientenakte - zusätzlich oder alternativ - auf einem Datenserver 3 als Datenspeicher gespeichert sein, wobei die dort, d.h. auf dem Datenserver 3, hinterlegte elektronische Patientenakte, umfassend die Patientendaten, unter der Kontrolle des Patienten 1 ist. Dabei sind die Patientendaten in dem Datenspeicher, insbesondere auf dem mobilen Datenkommunikationsgerät 2 bzw. auf dem Datenserver 3 verschlüsselt gespeichert und geschützt vor unbefugtem Zugriff. Eine Sammlung, Erweiterung, Bereitstellung und/oder Verwaltung der dort hinterlegten Patientendaten wird über das mobile Datenkommunikationsgerät 2 durch den Patienten 1 gesteuert. Ein Lesen, Freigeben und Speichern soll nur über den Patienten 1 möglich sein. Der Patient 1 sammelt und gibt seine Patientendaten über einen Informationscode 4, zum Beispiel über einen QR-Code, frei. Der Patient 1 kann einer Krankenkasse 5, in einem Krankenhaus 6, in einer Apotheke 7 und/oder einem Arzt 8 seine Patientendaten, oder vorzugsweise eine von ihm gewünschte Auswahl davon, zur Verfügung stellen.

Des Weiteren kann der Patient 1 seine persönlichen Gesundheitsdaten bzw. Patientendaten - zur Erweiterung und Aktualisierung seiner elektronischen Patientenakte - beispielsweise von dem Arzt 8 durch Scannen eines Informationscodes 4, den der Arzt 8 über eine entsprechende Computervorrichtung 9 mit geeigneter Anzeigeeinrichtung bereitstellt, erhalten. Für den Scanvorgang ist ein limitiertes Zeitfenster vorgesehen. Der Informationscode 4 umfasst die zu übertragenden Patientendaten. Des Weiteren kann in dem Informationscode 4 eine Adressinformation codiert sein, so dass der Patient 1 die mit der Adressinformation verknüpften Patientendaten über sein mobiles Datenkommunikationsgerät 2 herunterladen kann. Zudem kann der Patient 1 bestimmte Orte festlegen, an denen die Daten übertragen werden können. Somit erfolgt eine multimodale Kommunikation zum Austausch von persönlichen Patientendaten zwischen dem Patienten 1 und entsprechenden Einrichtungen oder behandelnden Ärzten.

Gemäß des in der einzigen Fig. dargestellten Ausführungsbeispiels haben Patienten die Möglichkeit ihre eigenen Patientendaten einfach vom Patientenverwaltungssystem bei einem Arzt 8 in ein dafür vorgesehenes Programm ihres Smartphones zu übernehmen. Das kann durch Scannen eines durch eine Computervorrichtung 9 mit geeigneter Anzeigeeinrichtung generierten und visualisierten patientenspezifischen Informationscodes 4, insbesondere in Form eines QR-Codes, erfolgen, wobei die Computervorrichtung 9 damit die entsprechenden Patientendaten aus dem Patientenverwaltungssystem beim Arzt 8 bereitstellt. Des Weiteren ist auch eine manuelle Eingabe der entsprechenden Informationen und Dokumente in das Smartphone möglich, falls der Arzt 8 keine geeignete Computervorrichtung 9 zur Codegenerierung oder zur Kommunikation mit dem Smartphone als mobiles Datenkommunikationsgerät 2 im Einsatz hat.

Somit hat der Patient die Behandlung sicher dokumentiert und die Daten immer zur Hand für weitere behandelnde Ärzte. Ärzte, die über eine geeignete Computervorrichtung 9 zum Datenaustausch mit dem Smartphone verfügen, können die Patientendaten einfach übernehmen. Ärzte erhalten somit einen Überblick über die vom Patienten individuell ausgewählten Informationen und können so zielgerichtet die Behandlung fortsetzen bzw. durchführen. Folglich werden teure und unnötige Doppeluntersuchungen und unpassende oder gar gefährliche Medikationen vermieden.

Hinsichtlich weiterer vorteilhafter Ausgestaltungen des erfindungsgemäßen Verfahrens bzw. des erfindungsgemäßen Systems wird zur Vermeidung von Wiederholungen auf den allgemeinen Teil der Beschreibung sowie auf die beigefügten Patentansprüche verwiesen.

Schließlich sei ausdrücklich darauf hingewiesen, dass das voranstehend beschriebene Ausführungsbeispiel des erfindungsgemäßen Verfahrens bzw. des erfindungsgemäßen Systems lediglich zur Erörterung der beanspruchten Lehre dient, diese jedoch nicht auf das Ausführungsbeispiel einschränken.

## Patentansprüche

1. Verfahren zum Betreiben einer elektronischen Patientenakte mit, vorzugsweise patientenspezifische bzw. -bezogene Informationen umfassenden, Patientendaten eines Patienten,
wobei die Patientendaten Text-, Bild-, Audio- und/oder Videodaten umfassen, wobei die Patientendaten in einem Datenspeicher unter der Kontrolle des Patienten gespeichert werden,
wobei die Patientendaten über ein mobiles Datenkommunikationsgerät gesammelt, bereitgestellt und/oder verwaltet werden, und
wobei der Patient einen Zugriff auf die elektronische Patientenakte, insbesondere zur Sammlung, Bereitstellung und/oder Verwaltung der Patientendaten, über das mobile Datenkommunikationsgerät steuert und/oder autorisiert.

2. Verfahren nach Anspruch 1, **dadurch gekennzeichnet, dass** als Datenspeicher zur Speicherung der Patientendaten ein lokal im mobilen Datenkommunikationsgerät angeordneter Speicher, ein vorzugsweise zentraler Server und/oder eine Cloud verwendet wird bzw. werden.

3. Verfahren nach Anspruch 1 oder 2, **dadurch gekennzeichnet, dass** das mobile Datenkommunikationsgerät eine Scaneinrichtung umfasst, wobei über die Scaneinrichtung Patientendaten für die elektronische Patientenakte erfasst werden.

4. Verfahren nach einem der Ansprüche 1 bis 3, **dadurch gekennzeichnet, dass** zur Sammlung von Patientendaten für die elektronische Patientenakte ein Informationscode, insbesondere ein QR-Code, verwendet wird, wobei der Informationscode von einer Patientendaten bereitstellenden Computervorrichtung, vorzugsweise für ein vorgebbares Zeitfenster, visualisiert wird, und wobei der Informationscode von einer von dem mobilen Datenkommunikationsgerät umfassten Scaneinrichtung gescannt wird.

5. Verfahren nach einem der Ansprüche 1 bis 4, **dadurch gekennzeichnet, dass** zur Bereitstellung von Patientendaten aus der elektronischen Patientenakte ein Informationscode, insbesondere ein QR-Code, verwendet wird, wobei der Informationscode von dem mobilen Datenkommunikationsgerät, vorzugsweise für ein vorgebbares Zeitfenster, visualisiert wird, und wobei der Informationscode von einer von einer Computervorrichtung umfassten Scaneinrichtung gescannt wird.

6. Verfahren nach einem der Ansprüche 1 bis 5, **dadurch gekennzeichnet, dass** der Informationscode Patientendaten des Patienten umfasst.

7. Verfahren nach einem der Ansprüche 1 bis 6, **dadurch gekennzeichnet, dass** der Informationscode eine Adressierungsinformation umfasst, wobei die Adressierungsinformation zur Adressierung eines Speicherorts mit hinterlegten Patientendaten dient, und wobei die an dem Speicherort hinterlegten Patientendaten mittels einer Datenübertragung, insbesondere über eine drahtlose Funkverbindung, abgerufen werden.

8. Verfahren nach einem der Ansprüche 1 bis 7, **dadurch gekennzeichnet, dass** bei der Sammlung von Patientendaten für die elektronische Patientenakte die Patientendaten in einem von der Computervorrichtung umfassten Datenspeicher als Speicherort zum Abrufen oder in einem von der Computervorrichtung kontrollierbaren Speicherbereich, vorzugsweise auf einem Server, als Speicherort zum Abrufen hinterlegt sind.

9. Verfahren nach einem der Ansprüche 1 bis 8, **dadurch gekennzeichnet, dass** bei der Bereitstellung von Patientendaten aus der elektronischen Patientenakte die Patientendaten in dem vom Patienten kontrollierten Datenspeicher als Speicherort zum Abrufen hinterlegt sind.

10. Verfahren nach einem der Ansprüche 1 bis 9, **dadurch gekennzeichnet, dass** die zum Abrufen hinterlegten Patientendaten für ein vorgebbares Zeitfenster, vorzugsweise ab Visualisierung des Informationscodes, zum Abrufen bereitgestellt werden.

11. Verfahren nach einem der Ansprüche 1 bis 10, **dadurch gekennzeichnet, dass** die Patientendaten, vorzugsweise über das Scannen des Informationscodes und/oder mittels einer Datenübertragung, in verschlüsselter Form auf das mobile Datenkommunikationsgerät übertragen werden, und wobei die verschlüsselten Patientendaten mit dem Datenkommunikationsgerät nur an einem vom Patienten zuvor festgelegten geographischen Ort entschlüsselt werden.

12. Verfahren nach einem der Ansprüche 1 bis 11, **dadurch gekennzeichnet, dass** das mobile Datenkommunikationsgerät vom Patienten derart konfigurierbar ist, dass der Zugriff auf die elektronische Patientenakte, insbesondere zur Sammlung, Bereitstellung und/oder Verwaltung der Patientendaten, über das mobile Datenkommunikationsgerät nur an einem vom Patienten zuvor festgelegten geographischen Ort durchführt wird.

13. Verfahren nach einem der Ansprüche 1 bis 12, **dadurch gekennzeichnet, dass** die aus der elektronischen Patientenakte bereitzustellenden Patientendaten und/oder die für die elektronische Patientenakte zu sammelnden Patientendaten durch den Patienten über das mobile Datenkommunikationsgerät selektiert werden.

14. Verfahren nach einem der Ansprüche 1 bis 13, **dadurch gekennzeichnet, dass** auf dem mobilen Datenkommunikationsgerät ein Programm installiert wird, wobei der Patient über das Programm die Patientendaten, vorzugsweise umfassend die patientenspezifischen bzw. -bezogenen Informationen, verwaltet.

15. System zum Betreiben einer elektronischen Patientenakte mit, vorzugsweise patientenspezifische bzw. -bezogene Informationen umfassenden, Patientendaten eines Patienten, insbesondere zur Durchführung eines Verfahrens nach einem der Ansprüche 1 bis 14,
wobei die Patientendaten Text-, Bild-, Audio- und/oder Videodaten umfassen, wobei das System einen Datenspeicher und ein mobiles Datenkommunikationsgerät umfasst,
wobei die Patientendaten in dem Datenspeicher unter der Kontrolle des Patienten speicherbar sind,
wobei die Patientendaten über das mobile Datenkommunikationsgerät gesammelt, bereitgestellt und/oder verwaltet werden, und
wobei ein Zugriff auf die elektronische Patientenakte, insbesondere zur Sammlung, Bereitstellung und/oder Verwaltung der Patientendaten, über das mobile Datenkommunikationsgerät durch den Patienten steuerbar und/oder autorisierbar ist.
